(19) **Europäisches Patentamt / European Patent Office / Office européen des brevets**

(11) **EP 4 574 100 A1**

(12) # EUROPEAN PATENT APPLICATION

(43) Date of publication:
**25.06.2025 Bulletin 2025/26**

(21) Application number: **23307330.3**

(22) Date of filing: **21.12.2023**

(51) International Patent Classification (IPC):
***A61F 2/91*** *(2013.01)*

(52) Cooperative Patent Classification (CPC):
**A61F 2/915;** A61F 2002/91508; A61F 2002/91558;
A61F 2230/001; A61F 2250/0039

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Université d'Aix-Marseille**
  **13007 Marseille (FR)**
• **Assistance Publique - Hôpitaux de Marseille**
  **13005 Marseille (FR)**
• **Ecole Centrale de Marseille**
  **13013 Marseille (FR)**
• **Centre National de la Recherche Scientifique**
  **75016 Paris (FR)**

(72) Inventors:
• **CHARPENTIER, Victor**
  **13004 MARSEILLE (FR)**
• **BOURGEOIS, Stéphane**
  **13012 MARSEILLE (FR)**
• **ALDEBERT, Philippe**
  **13008 MARSEILLE (FR)**
• **MARTHELOT, Joël**
  **13004 MARSEILLE (FR)**
• **ANDRADE SILVA, Ignacio Javier**
  **13002 MARSEILLE (FR)**

(74) Representative: **Cassiopi**
  **230, avenue de l'Aube Rouge**
  **34170 Castelnau-le-Lez (FR)**

(54) **EXPANDABLE STENT WITH A PROGRAMMABLE SHAPE AND/OR PROGRAMMABLE MECHANICAL PROPERTIES**

(57)  A stent comprising a body (10) with a wall (11) having a web structure configured to expand from a contracted configuration to an expanded configuration, the web structure comprising a plurality of cells (12), each cell (12) comprises beams connected together to form said cell (12), the geometry and dimensions of each cell (12) being configured to obtain a target shape and/or target mechanical properties of the stent (1) when the stent (1) passes from the contracted configuration to the expanded configuration upon the application of a predefined radially outward displacement on the stent (1).

Fig. 2

EP 4 574 100 A1

**Description**

The invention relates to an expandable stent, in particular a balloon expandable stent.

A stent is prosthetic device, tube-shaped, which is used for implantation in a body passageway (e.g. a lumen of a vein or of an artery) to keep the passageway open.

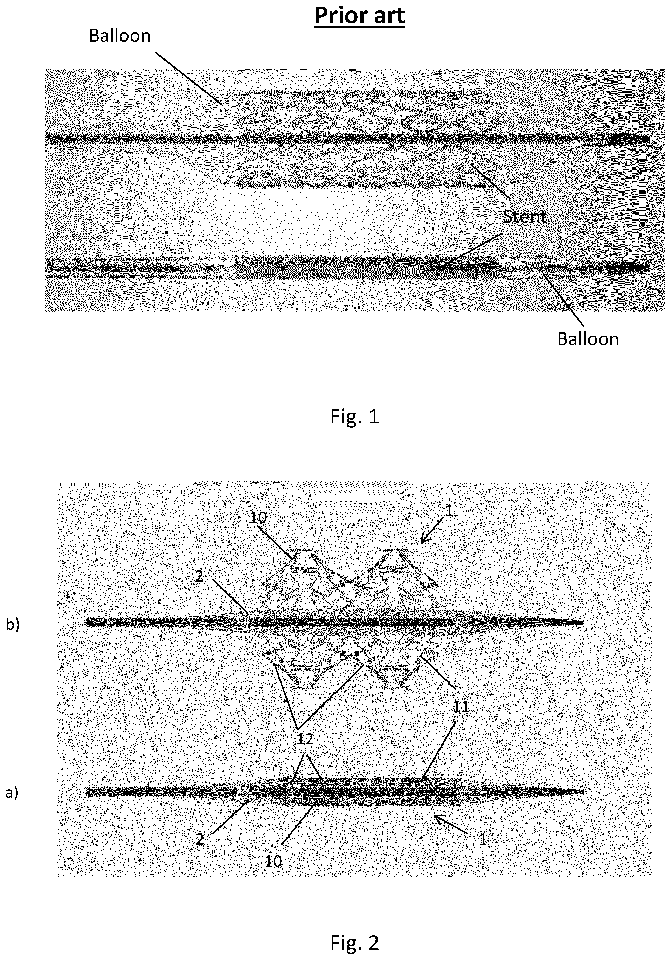

Known expandable stents (self-expanding or balloon-expandable) are configured to pass from an initial contracted configuration, in which the stent has a certain diameter, to an expanded configuration, in which said diameter increases. The expanded stents are usually tubular or cylindrical, meaning that their diameter is constant over the entire length of the stent. Figure 1 represents a balloon expandable stent of prior art in the contracted configuration and the expanded configuration.

However, as body passageways are not straight, it is difficult, even impossible, to treat some cardiovascular diseases or lesions. In fact, expanding straight stents (i.e. with a constant diameter) in a curved blood vessel can injure the body passageway. For example, it can cause delamination of the walls of the blood vessel and lead to the creation of aneurysms.

To try to overcome these drawbacks, at best, some stents of prior art have a variable flexibility along their length. In this context, EP 1 790 314 discloses an expandable stent comprising a body with a wall having intersecting members (or web structure). The intersecting members are arranged to define a plurality of repeating patterns. The flexibility and rigidity of the stent is varied along the length by varying the repeating pattern design, making it possible for the stent to curve. However, the stent curves only by being forced into a curve by the blood vessel in which it is inserted. Therefore the stent is disrupting the nominal form of the blood vessel in a non-controlled way.

Such a solution is thus not satisfactory to treat cardiovascular diseases or lesions.

The present invention aims at proposing an expandable stent which is able to deploy or expand in a non rectilinear manner (curved or flared) and which enables a variation of the radial force applied by the stent to the tissue to reduce springback in the area to be treated and preserve the surrounding healthy areas.

Accordingly, the invention provides a stent comprising a body with a wall having a web structure configured to expand from a contracted configuration to an expanded configuration, the web structure comprising a plurality of cells, the geometry and dimensions of each cell being configured to obtain a target shape and/or target mechanical properties of the stent when the stent passes from the contracted configuration to the expanded configuration upon the application of a predefined radially outward displacement on the stent.

In other words, the geometry and dimensions of each cell are independently configured to obtain the target shape and/or target mechanical properties when the stent passes from the contracted configuration to the expanded configuration upon the application of a predefined radially outward displacement on the stent. With yet other words, the geometry and dimensions of each cell are configured independently of those of the other cells.

By locally controlling the geometry and dimensions of each cell, the stent according to the present invention makes it possible to predict and control the shape of the stent once deployed/expanded, as well as the mechanical properties of the stent. The prediction of the shape and the mechanical properties is made locally (i.e. for each cell), making it possible to predict very accurately the shape and the mechanical properties of the entire stent.

The stent invention enhances the quality of care for patients suffering from congenital heart diseases which are currently untreated. It is for example possible to:

- have stiffness properties that change with stent length;
- treat lesions that are on curved vessels by providing a stent having a variable curvature;
- treat lesions on vessels that need the provision of a stent having a varying diameter along the length of the stent;
- provide re-expandable or multi-expandable stent with known mechanical properties at different opening times.

In one embodiment, the web structure comprises multiple parallel chains of cells bound together.

In one embodiment, each cell is configured to present a target Poisson's ratio when the stent passes from the contracted configuration to the expanded configuration.

In one embodiment, each cell is configured to present a unique target Poisson's ratio when the stent passes from the contracted configuration to the expanded configuration.

In one embodiment, each beam of each cell has dimensions which are predefined so as to obtain the target shape and/or the target mechanical properties.

In one embodiment, the length and the width of each beam of each cell are predefined so as to obtain the target shape and/or the target mechanical properties.

In one embodiment, the geometry of all the cells is identical, at least one cell having different dimensions from that of another cell.

In one embodiment, all the cells have the same overall length and overall width.

In one embodiment, the beams are parallel to one another when the stent is in the contracted configuration. Each cell comprises hinges and cross arms, each hinge linking a pair of said beams. Every two hinges and three beams form together an S-shape or a reverse S-shape. Each cross arm links a pair of S-shapes.

In one embodiment, each cell is symmetrical about a longitudinal median axis, said cell comprises two S-shapes, two reverse S-shapes and two cross arms.

In one embodiment, each cell comprises four beams and two hinges, the beams and the hinges forming a diamond shape in the contracted configuration.

In one embodiment, the stent is made of an elastoplastic material. Preferably, the stent is made of stainless steel.

Additional features and advantages of the present invention will become apparent from the subsequent description, taken in conjunction with the accompanying drawings, which show:

- figure 1 represents a balloon expandable stent of prior art, in a contracted configuration and an expanded configuration;
- figure 2 represents a balloon expandable stent according to one embodiment of the invention, in a contracted configuration and an expanded configuration;
- figure 3 shows a numerical model of the stent in the contracted configuration and the expanded configuration;
- figure 4 shows two examples of cells that may be used in the stent;
- figure 5 shows parameters which enable the variation of the mechanical properties and the shape of the cells;
- figure 6 shows an example of a plate with positive and negative Gaussian curvature using two cell geometries;
- figure 7 an example of shaping of a chain of cells;
- figure 8 represents at a) the behavior of an elastoplastic material and at b) cell shapes predicted by finite elements during extension;
- figure 9 shows models and finite element predictions for two symmetric cells;
- figure 10 shows models and finite element predictions for two asymmetric cells;
- figure 11 shows a model of discrete curvature of cells; and
- figure 12 shows an example of a programmed out-of plane shape.

Figure 2 shows an expandable stent 1 according to one embodiment of the invention, along with a balloon 2. Balloon 2 is inserted into stent 1.

This figure shows stent 1 in a contracted configuration at a), and in an expanded configuration at b). In figure 2a), balloon 2 is in the contracted configuration, meaning that balloon 2 is deflated. In figure 2b), balloon 2 is also represented in the contracted configuration.

Stent 1 comprises a body 10 with a wall 11 having a web structure. The web structure comprises a plurality of cells 12. The web structure comprises multiple parallel chains of cells 12. Cells 12 are bound together.

Wall 11 of stent 1 can be thus considered as discretized or meshed into a plurality of small elements, said small elements being cells 12.

Body 10 of stent 1 has a tubular shape in the contracted configuration. In the present document, a longitudinal direction corresponds to the direction of the axis of revolution of the tubular body.

Stent 1 is configured to expand from the contracted configuration to the expanded configuration upon the application of a radially outward displacement on stent 1 by balloon 2. For this purpose, starting from the contracted configuration, balloon 2 is inflated, pushing thus on an inner face of wall 11 and making stent 1 expand. The expanded configuration corresponds to the configuration wherein stent 1 is expanded and the radially outward displacement is no longer applied.

The geometry (i.e. the shape or pattern) and dimensions of each cell 12 are configured to obtain a target shape and/or target mechanical properties of the stent when the stent passes from the contracted configuration to the expanded configuration.

The radially outward displacement applied by balloon 2, prescribed by the characteristics of balloon 2, is considered in the design of stent 1 to obtain the target shape and/or target mechanical properties of stent 1 when stent 1 passes from the contracted configuration to the expanded configuration.

The target shape is set by setting a target Poisson's ratio for each cell 12 when stent 1 passes from the contracted configuration to the expanded configuration.

The geometry and dimensions of cells 12, and the radially outward displacement are predefined thanks to the knowledge of the behavior of the material chosen for stent 1, as explained in more details below.

Stent 1 is made of a biocompatible material. Preferably, stent 1 is made of stainless steel (e.g. stainless steel 316L). Alternatively, stent 1 can be made of cobalt chromium (e.g. cobalt chromium L-605), nickel cobalt (e.g. nickel cobalt MP35N), platinum chromium PtCr, or any other suitable biocompatible material.

The material of stent 1 is advantageously elastoplastic. This enables stent 1 in the expanded configuration to remain expanded and continue applying stresses on a body passageway in which it is inserted.

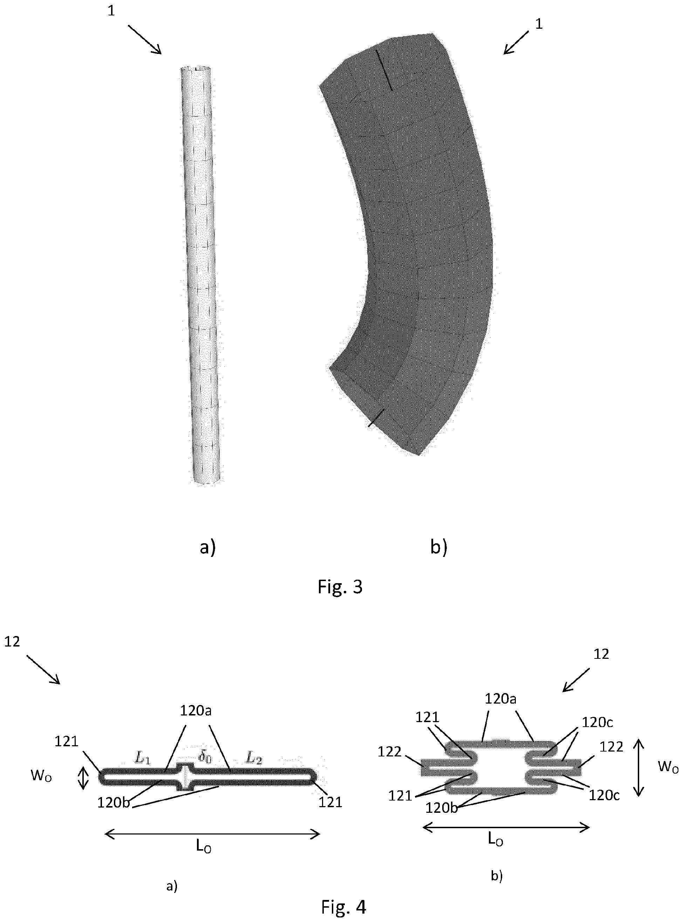

Figure 3 shows a numerical model of stent 1 in the contracted configuration at a), and in the expanded configuration at b).

The numerical model is generated by a simulation software, for example a finite element software. The simulation software enables the prediction of the behavior of stent 1 when it passes into the expanded configuration and to take into account the influence of the vasculature on stent 1. The numerical model helps validate the design of the stent before manufacturing.

The simulation software can be for example ABAQUS, ANSYS, SimScale or any other known simulation software using finite element method.

Figure 4 shows two examples of a cell's shape that may be used in stent 1. Cells 12 are here in the contracted configuration, i.e. before the application of any displacement. Of course, other shapes are also possible.

Each cell 12 comprises beams 120a, 120b, 120c connected together to form said cell.

Cells 12 can be closed lattice cells or open lattice cells. Figure 4a) shows a cell 12 having a diamond shape. This type of cells is a closed lattice cell because when pulled on, said cells contract a lot and have a high Poisson's ratio. Figure 4b shows a more complex shape and which is an open lattice cell. Open lattice cells refer to cells which, when pull on, the cells form a big opening compared to the closed lattice cells. The closed lattice cells have a greater Poisson's ratio than the open lattice cells.

The diamond cell 12 of figure 4a comprises four beams 120a, 120b and two hinges 121. Each hinge connects two beams 120a, 120b. The hinges 121 are here C-shaped arms.

The four beams 120a, 120b are here formed by two upper beams 120a and two lower beams 120b. The upper beams 120a are aligned with one another and lower beams 120b are aligned with one another, when stent 1 (and thus each cell 12) is in the contracted configuration. The upper beams 120a are parallel to lower beams 120b when the stent (and thus each cell) is in the contracted configuration. Each hinge 121 connects an upper beam 120a to a lower beam 120b.

The diamond cell of figure 4a is asymmetrical. Upper beams 120a have different lengths. One short upper beam 120a has a length L1 and one long upper beam 120a has a length L2 different of L1. Lower beams 120b have different lengths. One short lower beam 120b has a length L1 and one long lower beam 120b has a length L2 different of L1.

In another embodiment, cell 12 can be a diamond symmetrical cell. All four beams 120a, 120b can have the same length.

Cell 12 has an overall length $L_O$ corresponding in the embodiment of figure 4a to the distance between the hinges 121 or the sum of lengths L1 and L2. Cell 12 has an overall width $W_O$ corresponding in the embodiment of figure 4a to the distance between the upper beams 120a and the lower beams 120b.

Cell 12 of figure 4b comprises beams 120a, 120b, 120c, hinges 121 and cross arms 122. In particular, cell 12 comprises twelve beams 120a, 120b, 120c (two upper beams 120a, two lower beams 120b and eight intermediate beams 120c), eight hinges 121 and two cross arms 122. Of course, the number of beams, hinges and cross arms can be different.

Hinges 121 are here C-shaped arms or reverse C-shaped arms. In particular, cell 12 comprises four C-shaped arms and four reverse C-shaped arms.

Each hinge 121 links a pair of beams 120a, 120b, 120c. Every two hinges 121 and three beams 120a, 120b, 120c form together an S-shape or a reverse S-shape when the stent (and thus each cell) is in the contracted configuration. Cell 12 comprises two S-shapes and two reverse S-shapes. Each S-shape is here formed by three beams 120a, 120b, 120c and two hinges 121 (one C-shaped arm and one reverse C-shaped arm). Each reverse S-shape is here formed by three beams 120a, 120b, 120c and two hinges 121 (one C-shaped arm and one reverse C-shaped arm).

Each cross arm 122 links one of the S-shapes to one of the reverse S-shapes. Cross arm 122 is substantially perpendicular to the beams 120a, 120b, 120c when the stent (and thus each cell) is in the contracted configuration.

Cell 12 is here symmetrical about a longitudinal median axis, but can be of course asymmetrical.

Cell 12 has an overall length $L_O$ corresponding in the embodiment of figure 4b to the distance between the cross arms 122. Cell 12 has an overall width $W_O$ corresponding in the embodiment of figure 4b to the distance between the upper beams 120a and the lower beams 120b.

The geometry and dimensions of the cells, and the radially outward displacement applied on the inner face of the stent has an impact on the shape and the properties of the stent in the expanded configuration. The invention makes it possible, by tuning the local properties of each cell, to obtain the target shape and/or properties of the stent in the expanded configuration.

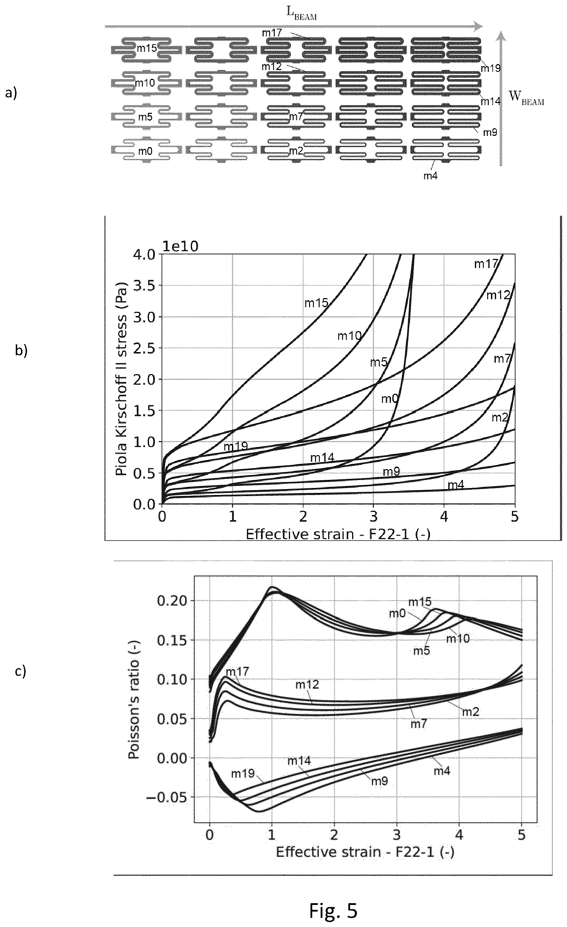

To illustrate this, figure 5 shows the parameters which enable the variation of the mechanical properties and the shape of cells 12. This figure presents results for the open lattice cell 12 of figure 4b provided as a non-limiting example. The same reasoning applies to other shapes of cells.

In figure 5a, the length $L_{beam}$ of the beams and the width $W_{beam}$ of the beams are varied. The width $W_{beam}$ of the beams shall mean the dimension of the beams in a transverse direction (here the longitudinal direction of the stent). The width of all the beams is varied equally in this example. The higher the width, the thicker it is represented on the figure.

Figure 5b shows, on the one hand, the stress-strain response of some of the cells of figure 5a as a function of the effective strain. In particular, figure 5b shows the stress-strain response of twelve cells m0, m2, m4, m5, m7, m9, m10, m12, m14, m15, m17, m19 having all the same shape but different length $L_{beam}$ and/or width $W_{beam}$ of the beams. It is noticed that the stress-strain response varies when varying the length $L_{beam}$ and the width $W_{beam}$ of the beams. The higher the length $L_{beam}$ of the beams, the lower the stress-strain response. The higher the width $W_{beam}$ of the beams, the higher the stress-strain response.

On the other hand, figure 5c shows Poisson's ratio for some of the cells of figure 5a as a function of the effective strain. In particular, figure 5b shows Poisson's ratio of cells m0, m2, m4, m5, m7, m9, m10, m12, m14, m15, m17, m19 having all the same shape but different length $L_{beam}$ and/or width $W_{beam}$ of the beams. By being able to predict Poisson's ratio of the cell, it is possible to predict its shape once expanded.

Thus, by controlling the length, the width and the displacement or strain applied on cells 12, it is possible to create a mesh that produces the desired shape (or target shape) of stent 1 in the expanded configuration. Based on the applied displacement and Poisson's ratio, an arrangement of cells 12 having a certain geometry and dimensions is created, said arrangement enabling the desired mechanical properties and/or shape of stent 1 to be achieved.

The method of the invention in order to obtain a target shape and/or target mechanical properties of stent 1 comprises the following steps:

- setting the target shape and/or target mechanical properties depending on the intended use; and
- mapping the arrangement of the cells in the contracted configuration in order to obtain the target shape and/or target mechanical properties at the passage to the expanded configuration.

The mapping step is made possible thanks to the knowledge of the mechanical properties of the material used to manufacture the stent. The mechanical properties are known through analytical models, as detailed below. Each cell is designed so as to achieve the local target shape and/or target mechanical properties. The mapping step is carried out taking into account the displacement applied on the stent by balloon 2. When balloon 2 is inflated, all cells 12 are subject to the same load, which is the opening by balloon 2. Balloon 2 has the same size throughout. The process to find the geometry and dimensions of each cell 12 is detailed below.

Once the mapping that enables the target shape and/or mechanical properties has been performed, stent 1 is manufactured according to the set arrangements of cells. Stent 1 can be produced by laser cutting.

In an embodiment of the invention, the stent is meshed such that all cells 12 have the same overall dimensions, namely the same overall length $L_O$ and the same overall width $W_O$. All cells 12 can have the same shape. The dimensions of beams 120a, 120b, 120c (in particular the length and/or the width of the beams) for each cell 12 are varied locally. Said otherwise, the dimensions of beams 120a, 120b, 120c are varied while maintaining constant the overall dimensions of cells 12. When balloon 2 is inflated, all cells 12 are subject to the same load, which is the opening by the balloon. Depending on the length's beams variation, different deformations are obtained in terms of extension for the same applied load. It is thus possible to predict the variation of the length along the stent, as well as the stent's diameter and curvature's variations.

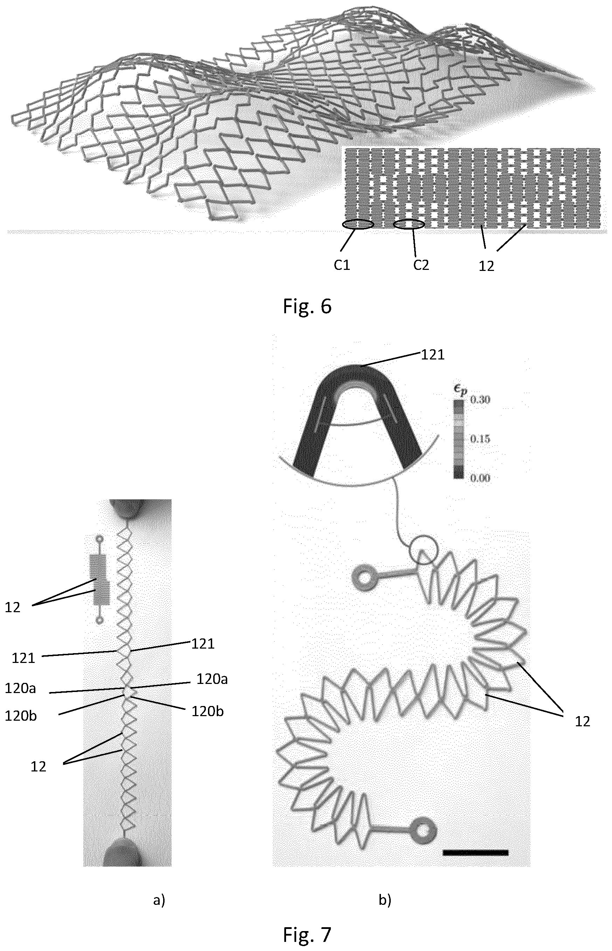

In the example of figure 6, all cells 12 have the same shape, namely the shape represented at figure 4b. The meshing of the stent is represented in the lower part of the figure. All the cells have the same overall dimensions (i.e. the same overall length $L_O$ and the same overall width $W_O$). Two types of cells 12 are used, namely a first type C1 and a second type C2. The length of the beams of first type C1 of cells is higher than the length of the beams of second type C2 of cells. The overall dimensions of both types C1, C2 are the same, as said before. The specific shape of cells 12 used allows varying the opening and recovery (or springback) distance of each cell in the longitudinal direction, and the way the cells retract in the transverse direction. Said otherwise, the cells do not deform in the same way in both longitudinal and transverse directions once the applied load/force is released. The deformed cells are represented on the figure, showing positive bumps and negative bumps created thanks to the shape of the cells and the specific meshing. Of course, other shapes of cells can result in the same effect.

To validate the method of the invention for finding the target shape and/or target mechanical properties, analytical models are injected into a simulation software. Numerical models are generated by the simulation software. The simulation software is typically a finite element software. The simulation software enables the prediction of the behavior of an element (typically the stent) under the effect of a load. The simulation software can be for example ABAQUS, ANSYS, SimScale or any other known simulation software using finite element method. Also, experimental tension tests for assemblies with parallel chains of cells. Comparison is made between the experimental tension tests, the analytical models and the numerical models in order to validate the method. This process is explained and illustrated with examples hereafter.

An illustration of the approach

Figure 7 shows an example of a chain of cells 12. The chain of cells is typically 3D printed for the experimental test. The chain of cells comprises diamond cells (as in the example of figure 4a). Each cell 12 comprises four beams 120a, 120b connected by hinges 121. Cells 12 are here asymmetrical. Upper beams 120a have different lengths from one another and lower beams 120b have different lengths from one another.

As shown in figure 7a), the chain of cells is stretched by a factor $\lambda = \delta/\delta_0$, where $\delta_0$ is the initial transverse distance between the upper beams and the lower beams, and $\delta$ is said transverse distance after stretching. The stretching causes plastic deformation of the structure. When relaxed, the chain of cells adopts a curved shape at equilibrium. The curvature of the shape at equilibrium is programmed from the asymmetry of cells 12 along the chain to form two semicircles of opposite curvature.

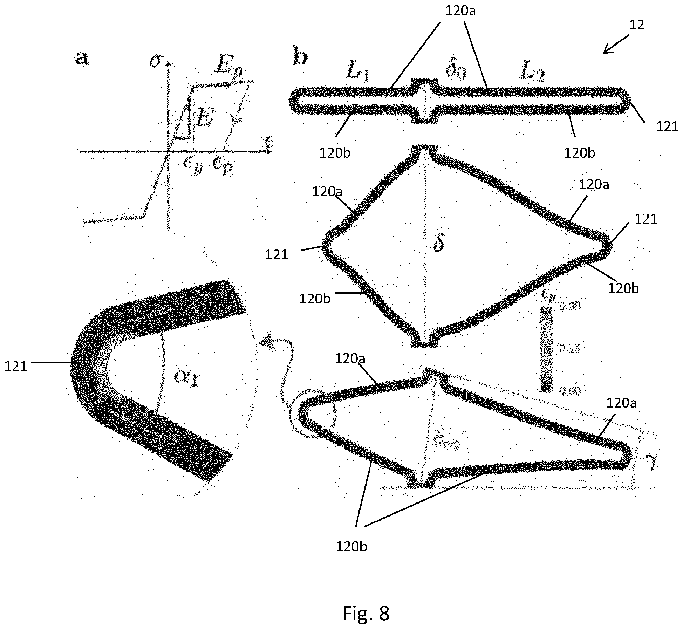

The material used in this experience is polyethylene terephthalate glycol-modified (PETG), which is suitable for 3D printing. As can be seen in figure 8, this material is modeled as a bilinear elastoplastic material with Young modulus E, yield strain $\varepsilon_Y$ and tangent modulus $E_p$.

As evident from the numerical model of figure 8, the beams act as lever arms that concentrate bending moment in the hinges. The hinge of the shorter beam stores more plastic deformation, which results in a larger equilibrium angle $\alpha_1$. This asymmetry has two origins: (i) basic geometry enforces that the opening angle of the shorter beams is larger than that of the longer ones; and (ii) the long beams bend more than the short ones, thereby amplifying this disparity. The deformation in the hinges is plastic, so that equilibrium angles $\alpha 1$ and $\alpha 2$ differ when the boundaries are released, breaking the system's symmetry and causing the cell to tilt with angle $\gamma$ and length $\delta_{eq}$. Stacking cells together thus allows to locally control the discrete curvature $\kappa \sim \gamma/\delta_{eq}$ and thus obtain arbitrarily complex shapes in the plane.

The models that allow solving the inverse design problem are detailed hereafter.

<u>Deformation of a chain of symmetric cells</u>

First, the deformation of a chain of symmetric cells is considered.

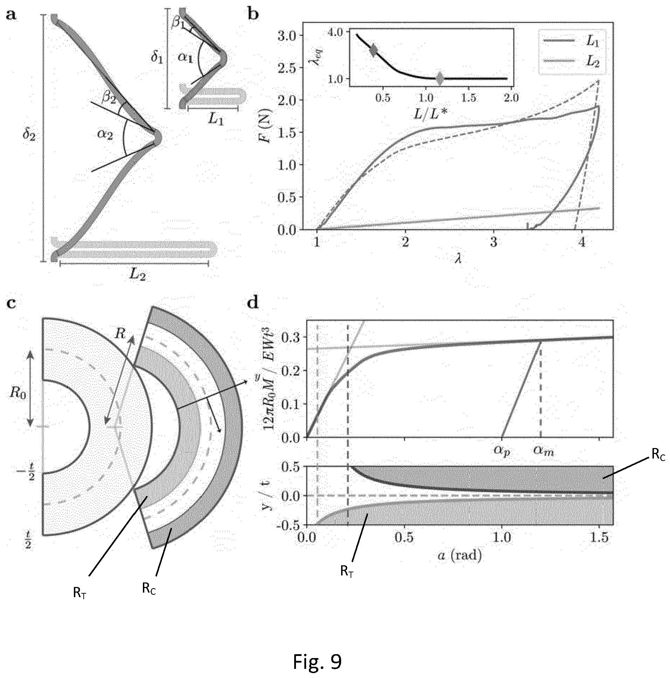

Figure 9a shows the finite element predictions of the deformed configuration of two symmetric cells: one of the cells has beams with a length L1 = 4 mm and the other cell has beams with a length L2 = 12 mm. Both cells have a thickness t = 0.4 mm and an initial radius $R_0$ = 0.4 mm. Under periodicity assumptions, the kinematics are fully characterized by the hinge angle $\alpha$ and the bending angle $\beta$. The prediction of the model is overlaid on the finite element results showing good agreement.

Figure 9b shows the force displacement curves for the two different geometries of cells in figure 9a for a stretching factor $\lambda$ = 4.2. Experiments are represented by solid lines and model predictions by dotted lines. This figure also shows the equilibrium cell stretch (or residual stretch) $\lambda_{eq}$ for a maximum stretch $\lambda$ = 4.2 as a function of the ratio of the beam length over a critical length $L^*$ separating the elastic and plastic regimes.

The force recorded in the experiments for the short beam (L1 = 4 mm) first increases linearly with displacement before reaching a plateau. When the stress is released, the cell is permanently deformed with the residual stretch $\lambda_{eq}$. For the same imposed displacement, cells with longer beams (L2 = 12 mm) stay in the elastic regime with no permanent deformation.

To predict the mechanical response of the cell as a function of the problem's parameters, it is assumed that the hinges deform while remaining circular (figure 9c), such that the strain across the thickness of the central hinge can be calculated as follows:

$$\epsilon(y, \kappa) = \frac{y(\kappa - \kappa_0)}{1 + \kappa_0 y}$$

or,

$$\epsilon(\alpha, y) = -\frac{y\alpha}{\pi(R_0 + y)}$$

where $\alpha$ is the hinge angle, $R_0$ the radius of curvature and y the coordinate through the thickness.

As the hinge opens, plasticity first appears below the neutral line in areas that are in tension (region $R_T$ shown in figure 9c). For larger values of $\alpha$, plastic deformations also occur above the neutral line in areas in compression (region Rc in figure 9c). Therefore, the hinge moment $M(\alpha) = \int_{-t/2}^{t/2} y\sigma\big(\epsilon(y, \alpha)\big) dy$ is calculated analytically in three regimes indicated by the dotted lines in figure 9d: fully elastic, with a single tensile plasticized region, with two plasticized regions. It is noticed that the system behaves as a piecewise linear torsional spring, with transitions that match the three aforementioned regimes as indicated by the change in slope in figure 9d. When the cell is released, the hinge angle relaxes elastically from its maximum value $\alpha_m$ down to a new equilibrium position defined by $\alpha_p$ following the slope obtained in the elastic regime.

On the other hand, the bending energy of the beam that connects the hinges depends on its flexural rigidity and

the angle $\beta$. In fact, the beams are modeled as Euler-Bernoulli beams. It is defined the deflection of the beam $\omega$ with respect to a coordinate system rotated by an angle $\alpha/2$ with respect to the x-axis. This beam is, therefore, considered as one whose ends are pinned along this rotated axis and whose two ends are at an angle $-\beta$ to the rotated axis:

$$EI\omega^{(4)}(x) = 0, \text{ with } I = Wt^3/12.$$

Integrating this equation with boundary conditions $\omega\left(\pm\frac{L}{2}\right) = -\tan\beta$ and the bending energy stored in one arm is:

$$\frac{1}{2}EI\int_{-L/2}^{L/2}(\omega''(x))^2 dx = E\frac{Wt^3}{2L}\tan^2\beta$$

No plasticity is developed in the beams. The geometry constrains the relationship between the two angles and the height (or width or transverse dimension) of the cell. Assuming that the shortening of the beams due to bending is small:

$$\delta = 2L\sin\left(\frac{\alpha}{2}+\beta\right) + 4R_0\frac{\pi}{\pi-\alpha}\cos(\frac{\alpha}{2})$$

The energy of the cell is expressed using a Lagrangian formulation:

$$L(\alpha,\beta) = 2\int_0^\alpha M(\alpha')\,d\alpha' + E\frac{Wt^3}{L}\tan^2\beta + F(\delta$$
$$-\left(2L\sin\left(\frac{\alpha}{2}+\beta\right) + 4R_0\frac{\pi}{\pi-\alpha}\cos\left(\frac{\alpha}{2}\right)\right))$$

where F is a Lagrangian multiplier that corresponds to the axial force, with

$$\frac{dL}{d\alpha} = 0,$$

$$\frac{dL}{d\beta} = 0.$$

The system's energy is then minimized, yielding an implicit kinematic relationship between $\alpha$ and $\beta$:

$$M(\alpha) = E\frac{Wt^3 sec^2\beta \tan\beta}{L^2\cos\left(\frac{\alpha}{2}+\beta\right)}\left(L\cos\left(\frac{\alpha}{2}+\beta\right) - \frac{2\pi R_0}{\pi-\alpha}\left(\sin\left(\frac{\alpha}{2}\right) - \frac{2\cos\left(\frac{\alpha}{2}\right)}{\pi-\alpha}\right)\right)$$

This equation is solved numerically.
To validate this model, the shape predicted by the analytical model is superimposed onto the numerical calculation in figure 9a. The model also predicts force as a function of displacement plotted as a dashed line in figure 9b in good agreement in experiments. In the inset of figure 9b, the prediction of the residual stretch $\lambda_{eq}$ is reported as a function of the beam lengths. It is observed a transition between permanently deformed cells for short beams and elastic regime for longer beams for the critical length $L^*$ which depends on $\lambda$, $\varepsilon_Y$, and geometry. The critical length $L^*$ can be written as:

$$L^* \sim 2R_0 \sqrt{\frac{2\Phi(\lambda - 1)}{\epsilon_Y} - \frac{l_e}{2}}$$

where $l_e$ is the elastic origami length and equal to $\pi t^3/(4R_0 b)$.

Mechanical response of asymetric cells

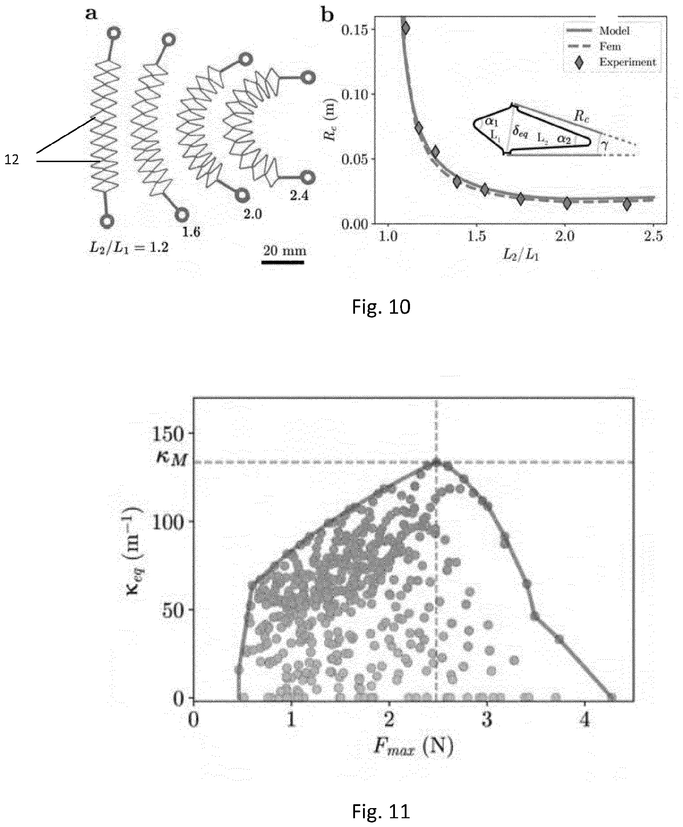

Asymmetric cells are now considered at figure 10. In particular, figure 10a shows cells comprising two different beam lengths L1 and L2 are considered. As can be seen in the figure, a variation of ratio L2/L1 leads to a very important variation of the radius of curvature $Rc$ of the chain of asymmetric cells.

Figure 10b shows the radius of curvature $Rc$ of the deformed chain as a function of beam lengths L2/L1 for $\lambda = 4.2$. Pulling and releasing such a cell yields a tilted shape with angle $\gamma$, a joint-to-joint distance $\delta_{eq}$, and discrete radius of curvature $Rc = \delta_{eq} /(2 \sin(\gamma/2))$.

The cell's energy is minimized to predict the value of $Rc$. The Lagrangian formulation of the energy of asymmetric cell can be indeed written as:

$$L_C(\alpha_1, \alpha_2, \gamma) = k_{el}((\alpha_1 - \alpha_{1,p})^2 + (\alpha_2 - \alpha_{2,p})^2 + (\alpha_1 + \gamma - \alpha_{1,p})^2$$
$$+ (\alpha_2 + \gamma - \alpha_{2,p})^2) + \Lambda C(\alpha_1, \alpha_2, \gamma)$$

where $\Lambda$ is a Lagrange multiplier and $C(\alpha_1, \alpha_2, \gamma) = 0$ is a geometrical constraint that links the angles $\alpha_1$, $\alpha_2$ and the tilt angle $\gamma$.

The minimization of this equation gives $\alpha_1$, $\alpha_2$ and $\gamma$ at equilibrium and $\delta_{eq}$ is computed geometrically.

The prediction for $Rc$ is shown in figure 10b, compared to experiments and full-fledged simulations. The figure shows that $Rc$ depends solely on the beam length ratio, thereby helping in solving the inverse design problem as detailed next.

Process to find the geometry and dimensions of a chain of cells

The process to find the geometry and dimensions of a chain of cells, which will morph toward a target chain shape when subject to loading is the following.

The target shape is first discretized into vertices and edges with uniform length $\delta_{eq}$ and variable angle $\gamma$.

Each pair $\delta_{eq,\gamma}$ is mapped to that of an asymmetric unit cell and used to calculate the discrete curvature of the cell at equilibrium $\kappa = 1/R_c$.

The cell's beams lengths $L1$ and $L2$, and the force $F$ that needs to be applied are found using an envelope of the discrete curvature of cells $\kappa$ shown in figure 11, which is generated using a model for a range of values of beam lengths. More precisely, figure 11 represents the discrete curvature of cells ($\kappa = 1/Rc$) as a function of the force $F$ applied during extension for all combinations of $(L1; L2) \in [2; 15]$ mm $\times$ [2; 15] mm.

Achieving a target three-dimensional shape

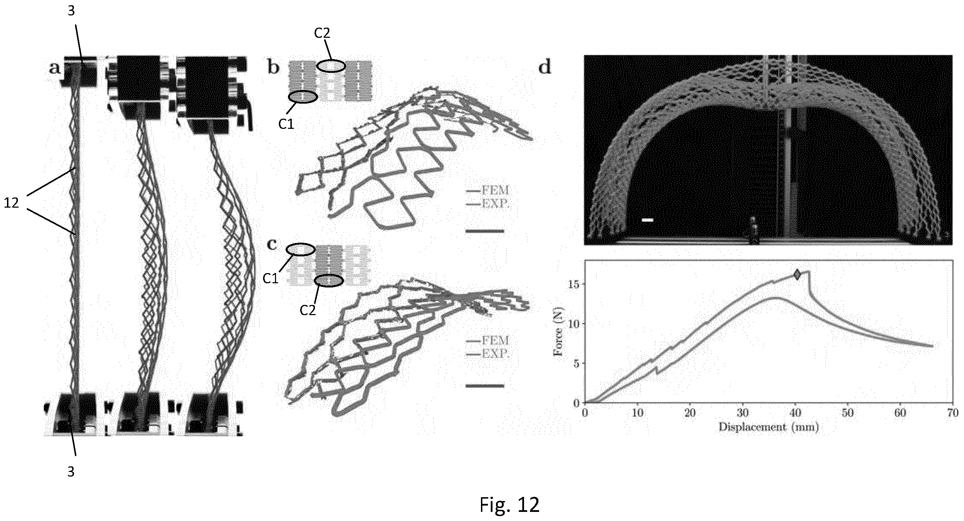

The process according to the invention to obtain a target three-dimensional shape when multiple parallel chains are bound together is the following. It is considered a structure with at least two chains of cells 12. The chains of cells are designed to return to a different length when actuated. The cells are for example symmetric and therefore have no intrinsic curvature. However, geometrical incompatibilities originating from their length mismatch cause the entire structure to bend out-of-plane (as can be seen in figure 12).

Figure 12a shows an experimental test wherein the relaxation of a plate-like assembly made of a plurality of chains of cells from planar at $\delta max$ to out-of-plane at $\delta eq$. The boundaries of the initial chain of cells are fixed to clamps 3 composed of sliders that allow free movement perpendicular to the tensile axis. When the boundaries are relaxed, the structure deforms with a positive Gaussian curvature (figure 12a).

The minimal grid for observing positive (Fig. 12b) and negative (Fig. 12c) Gaussian curvature is composed of three rows or chains of cells. As visible in figures 12b and 12c, two types of cells C1, C2 are used which are the same as in figure 6. Two chains of cells C1 are used in figure 12b with a chain of cells C2 between said two chains of cells C1. Two chains of cells C2 are used in figure 12c with a chain of cells C1 between said two chains of cells C2.

The experimental shapes are 3D scanned and compared to direct finite element simulations, showing excellent agreement. An estimate of the Gauss curvature of the resulting membrane is obtained by considering the gradients of in-plane strain. The transversal gradient of the axial strain yields $K_g \approx -\partial^2 \overline{\varepsilon_{yy}}/\partial x^2$. This value is obtained for each cell in isolation: $\overline{\varepsilon_{yy}} = (\delta_{eq} - \delta_{max})/\delta_{max}$, where $\delta_{max}$ is the maximum extension during traction. The transversal gradient of the axial strain obtained is then $K_g \approx -(\varepsilon_{yy}^{(+1)} + \epsilon_{yy}^{(-1)} - 2\epsilon_{yy}^{(0)})/W_c^2$, where the superscripts refer to the row index and $W_c$ is the cell's width. This simple prediction ($K_g = XX$ and $K_g = XX$) gives a good guideline for predicting the necessary arrangement of cells in the grid.

Discrete bits of Gaussian curvature can be distributed at specific position on a plane. More complex shapes consisting of discrete patch of positive and negative curvature leads to bridge structures.

More complex shapes composed of grids deploying towards shapes consisting of discrete curvature can be obtained by varying the cell structure along the length of the grid (figure 12d).

Generalization

In the examples of figures 7-12, only designs where plastic deformation is confined to the hinges and beams, which deform in bending have been considered. To enrich the design space, this constraint can be relaxed and cells where plastic deformation can be localized over the entire beam can be considered. To predict the mechanical response of these beams, a general Kirchhoff rod centerline model for an elastoplastic material is introduced. Deformation depends on the beam's rest curvature, which is updated during plastic deformation. This model can predict the force response of more complicated cells' shapes.

Thanks to its design, the stent according to the invention enables the programming of its shape and/or mechanical properties once expanded. This programmable stent can be adapted to specific patient geometries. In particular, the invention makes it possible to produce a stent with a non-rectilinear shape. Four main applications have been identified, covering the majority of pathologies:

- Curved stent for the treatment of coarctation of the aorta;
- Hourglass-shaped stent for the treatment of atrial septal defect;
- Diameter-reducing hourglass stent for percutaneous pulmonary valve replacement; and
- Re-expandable or multi-expandable stents.

**Claims**

1. A stent comprising a body (10) with a wall (11) having a web structure configured to expand from a contracted configuration to an expanded configuration, the web structure comprising a plurality of cells (12), each cell (12) comprises beams (120a, 120b, 120c) connected together to form said cell (12), the geometry and dimensions of each cell (12) being configured to obtain a target shape and/or target mechanical properties of the stent (1) when the stent (1) passes from the contracted configuration to the expanded configuration upon the application of a predefined radially outward displacement on the stent (1).

2. The stent according to claim 1, wherein the web structure comprises multiple parallel chains of cells (12) bound together.

3. The stent according to claim 1 or claim 2, wherein each cell (12) is configured to present a target Poisson's ratio when the stent (1) passes from the contracted configuration to the expanded configuration.

4. The stent according to claim 3, wherein each cell (12) is configured to present a unique target Poisson's ratio when the stent (1) passes from the contracted configuration to the expanded configuration.

5. The stent according to any one of claims 1 to 4, wherein the dimensions of each beam (120a, 120b, 120c) of each cell (12) being predefined so as to obtain the target shape and/or the target mechanical properties.

6. The stent according to claim 5, wherein the length and the width of each beam (120a, 120b, 120c) of each cell (12) being predefined so as to obtain the target shape and/or the target mechanical properties.

7. The stent according to any of claims 1 to 6, wherein the geometry of all the cells (12) is identical, at least one cell (12) having different dimensions from that of another cell (12).

8. The stent according to any of claims 1 to 7, wherein all the cells (12) have the same overall length and overall width.

9. The stent according to any one of claims 1 to 8, wherein the beams (120a, 120b, 120c) are parallel to one another when the stent (1) is in the contracted configuration, each cell (12) comprising hinges (121) and cross arms (122), each hinge (121) linking a pair of said beams (120a, 120b, 120c), every two hinges (121) and three beams (120a, 120b, 120c) form together an S-shape or a reverse S-shape, each cross arm (122) linking a pair of S-shapes.

10. The stent according to claim 9, wherein each cell (12) is symmetrical about a longitudinal median axis, said cell comprises two S-shapes, two reverse S-shapes and two cross arms.

11. The stent according to any one of claims 1 to 8, wherein each cell (12) comprises four beams (120a, 120b) and two hinges (121), the beams (120a, 120b) and the hinges (121) forming a diamond shape in the contracted configuration.

12. The stent according to any of claims 1 to 11, being made of an elastoplastic material, preferably of stainless steel.

# Prior art

Balloon

Stent

Balloon

Fig. 1

Fig. 2

a)                                    b)

Fig. 3

a)                                    b)

Fig. 4

Fig. 5

Fig. 6

a)                    b)

Fig. 7

Fig. 8

Fig. 9

Fig. 10

Fig. 11

Fig. 12

# EUROPEAN SEARCH REPORT

**Application Number**

EP 23 30 7330

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/015231 A1 (SACHAR RAVISH [US] ET AL) 17 January 2019 (2019-01-17) * paragraphs [0076], [0283], [0849]; figures 4a,17a-24 * | 1-12 | INV. A61F2/91 |
| X | WO 2019/033121 A1 (ELIXIR MEDICAL CORP [US]) 14 February 2019 (2019-02-14) * paragraphs [0005], [0032], [0033]; figure 1 * | 1-12 | |
| X | US 2016/235895 A1 (COSTELLO KIERAN [IE]) 18 August 2016 (2016-08-18) * paragraphs [0053], [0054]; figures 13a-13d * | 1-12 | |

TECHNICAL FIELDS
SEARCHED      (IPC)

A61F

The present search report has been drawn up for all claims

| Place of search | Date of completion of the search | Examiner |
|---|---|---|
| The Hague | 24 May 2024 | Espuch, Antonio |

## ANNEX TO THE EUROPEAN SEARCH REPORT
## ON EUROPEAN PATENT APPLICATION NO.

EP 23 30 7330

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2024

| Patent document cited in search report | Publication date | Patent family member(s) | Publication date |
|---|---|---|---|
| US 2019015231 A1 | 17-01-2019 | AU 2016344149 A1 | 17-05-2018 |
| | | AU 2020260431 A1 | 26-11-2020 |
| | | AU 2022202705 A1 | 19-05-2022 |
| | | CN 108289731 A | 17-07-2018 |
| | | CN 112043475 A | 08-12-2020 |
| | | EP 3367967 A1 | 05-09-2018 |
| | | HK 1258129 A1 | 08-11-2019 |
| | | JP 7075122 B2 | 25-05-2022 |
| | | JP 7368877 B2 | 25-10-2023 |
| | | JP 2018531751 A | 01-11-2018 |
| | | JP 2022106915 A | 20-07-2022 |
| | | US 2017112647 A1 | 27-04-2017 |
| | | US 2019015231 A1 | 17-01-2019 |
| | | US 2021228386 A1 | 29-07-2021 |
| | | US 2022378591 A1 | 01-12-2022 |
| | | US 2022378592 A1 | 01-12-2022 |
| | | WO 2017075273 A1 | 04-05-2017 |
| WO 2019033121 A1 | 14-02-2019 | CN 111212617 A | 29-05-2020 |
| | | CN 112773583 A | 11-05-2021 |
| | | CN 114983642 A | 02-09-2022 |
| | | EP 3664752 A1 | 17-06-2020 |
| | | JP 7220201 B2 | 09-02-2023 |
| | | JP 2020530359 A | 22-10-2020 |
| | | JP 2021180913 A | 25-11-2021 |
| | | JP 2023134672 A | 27-09-2023 |
| | | WO 2019033121 A1 | 14-02-2019 |
| US 2016235895 A1 | 18-08-2016 | US 2013103162 A1 | 25-04-2013 |
| | | US 2016235895 A1 | 18-08-2016 |
| | | WO 2013062946 A1 | 02-05-2013 |

For more details about this annex : see Official Journal of the European Patent Office, No. 12/82

**EP 4 574 100 A1**

**Patent documents cited in the description**

- EP 1790314 A